# Europäisches Patentamt
## European Patent Office
## Office européen des brevets

(19)

(11) Veröffentlichungsnummer: **0 069 697**

**A2**

(12) **EUROPÄISCHE PATENTANMELDUNG**

(21) Anmeldenummer: 82730088.0

(22) Anmeldetag: 25.06.82

(51) Int. Cl.³: **C 12 N 15/00**
C 12 P 13/24, C 12 N 1/20
//C12R1/19

(30) Priorität: 08.07.81 DE 3127361

(43) Veröffentlichungstag der Anmeldung:
12.01.83 Patentblatt 83/2

(84) Benannte Vertragsstaaten:
AT BE CH DE FR GB IT LI LU NL SE

(71) Anmelder: SCHERING AKTIENGESELLSCHAFT Berlin
und Bergkamen
Müllerstrasse 170/178 Postfach 65 03 11
D-1000 Berlin 65(DE)

(72) Erfinder: Siewert, Gerhard, Dr.
Im Fischgrund 50c
D-1000 Berlin 28(DE)

(72) Erfinder: Boidol, Werner
Schaperstrasse 19
D-1000 Berlin 15(DE)

(54) Herstellung und Anwendung von Plasmiden mit Genen für die Biosynthese von L-Prolin.

(57) Die vorliegende Erfindung betrifft die Herstellung von Plasmiden, die Gene für die Biosynthese von L-Prolin tragen, wobei eines dieser Gene so mutiert ist, daß die normalerweise vorhandene allosterische Hemmung der Biosynthese dieser Aminosäure entfällt.

Die Erfindung betrifft ferner die Anwendung dieser Plasmide zur Entwicklung von Bakterienstämmen der Art Escherichia coli, die L-Prolin in die Kulturflüssigkeit ausscheiden und daher für die fermentative Herstellung dieser Aminosäure besonders geeignet sind. Dabei können die Plasmide in den Zellen die Fähigkeit zur Überproduktion von L-Prolin entweder überhaupt erst bewirken oder eine bereits vorhandene, chromosomal bedingte Überproduktionsfähigkeit erheblich steigern.

Außerdem ist noch die Herstellung von geeigneten Wirtszellmutanten, die in Verbindung mit diesen Plasmiden eine besonders hohe Konzentration von L-Prolin in der Kulturflüssigkeit hervorbringen, Gegenstand der vorliegenden Erfindung.

EP 0 069 697 A2

1

Beschreibung

Die vorliegende Erfindung betrifft die Herstellung von Plasmiden, die Gene für die Biosynthese von L-Prolin tragen, wobei eines dieser Gene so mutiert ist, daß die normalerweise vorhandene allosterische Hemmung der Biosynthese dieser Aminosäure entfällt.

Die Erfindung betrifft ferner die Anwendung dieser Plasmide zur Entwicklung von Bakterienstämmen der Art Escherichia coli, die L-Prolin in die Kulturflüssigkeit ausscheiden und daher für die fermentative Herstellung dieser Aminosäure besonders geeignet sind. Dabei können die Plasmide in den Zellen die Fähigkeit zur Überproduktion von L-Prolin entweder überhaupt erst bewirken oder eine bereits vorhandene, chromosomal bedingte Überproduktionsfähigkeit erheblich steigern.

Außerdem ist noch die Herstellung von geeigneten Wirtszellmutanten, die in Verbindung mit diesen Plasmiden eine besonders hohe Konzentration von L-Prolin in der Kulturflüssigkeit hervorbringen, Gegenstand der vorliegenden Erfindung.

Die Biosynthese von L-Prolin aus L-Glutaminsäure wird in Escherichia coli sowie in verschiedenen anderen Mikroorganismen durch die Enzyme γ-Glutamyl-Kinase (Gen proB), γ-Glutamylphosphat-Reductase (Gen proA) und 1-Pyrrolin-5-carboxylat-Reductase (Gen proC) katalysiert /D.J. Hayzer und Th. Leisinger, J.Gen.Microbiol. 118, 287-293 (1980)/. Überschüssiges L-Prolin bewirkt sowohl eine allosterische Hemmung des ersten Enzyms als auch eine Repression der Biosynthese der beiden ersten Enzyme dieser Reaktionsfolge, wodurch eine Überproduktion der Aminosäure in Wildtypzellen verhindert wird /H.E. Umbarger, Ann. Rev. Biochem. 47, 533-606 (1978)/.

Es sind Mutanten verschiedener Escherichia coli-Stämme bekannt, die gegen mehrere Prolin-Antimetaboliten, z.B. 3,4-Dehydro- D/L-Prolin oder Azetidin-2-carbonsäure, resistent sind und die geringe Mengen L-Prolin in die Kulturflüssigkeit ausscheiden.

2

A. Baich und D.J. Pierson, Biochem. Biophys. Acta 104, 397-404 (1965), beschreiben z.B. eine Mutante des Stammes E.coli W, die ca. 250-350 mg/l L-Prolin in einem Medium mit 0,5 % Glucose und 0,1 % Glutaminsäure produziert. Diese Stämme sind offenbar in dem Gen proB so mutiert, daß die entsprechende γ-Glutamyl-Kinase durch L-Prolin nicht mehr allosterisch gehemmt wird /H. Condamine, Ann. Inst. Pasteur 120, 126-143 (1971)/.

Es wurde nun gefunden, daß durch Klonierung von chromosomalen DNS-Fragmenten, die aus antimetabolitresistenten Mutanten der oben beschriebenen Art isoliert wurden und die die dicht benachbarten Gene proA und proB tragen, neukombinierte Plasmide erhalten werden können, welche die Fähigkeit zur Ausscheidung von L-Prolin auf zahlreiche E.coli-Stämme, die von sich aus keine Überproduktion von L-Prolin aufweisen, übertragen können. Auf diesen Plasmiden liegt das Gen proB in der mutierten Form vor, was den Verlust der allosterischen Hemmung zur Folge hat (hier als Gen proB(mut.) bezeichnet).Plasmide dieser Art sind die in Beispiel 1 beschriebenen pSB121, pSB124 und pSB125. Ihre Eignung zur Steigerung der Ausscheidung von L-Prolin in Stämmen, die diese Aminosäure auch ohne Plasmid (chromosomal bedingt) ausscheiden, wird anhand der Tabelle 1 in Beispiel 3 belegt.. (pSB125 ergab die gleichen Werte wie pSB124).

Gegenstand der Erfindung ist zunächst die Verwendung der Mutante E.coli SB1 zur Herstellung des Plasmids pSB121 sowie die Verwendung des Plasmids pSB121 zur Herstellung der Plasmide pSB122, 123, 124, 125 und 126.

Die Erfindung betrifft ferner Plasmide, die eine DNS-Sequenz mit den Genen proA und proB enthalten, wobei proB in einer mutierten Form vorliegt, die eine antimetabolitresistente γ-Glutamyl-Kinase codiert.

Die Fähigkeit zur Überproduktion von L-Prolin kann weiter gesteigert werden, wenn man mit Hilfe der Methoden zur in-vitro-Neu-

kombination von DNS Plasmide hergestellt, die außer proA und proB (mut.) auch das Gen proC tragen. pSB122, pSB123 und pSB126, deren Herstellung in Beispiel 2 beschrieben wird, repräsentieren diesen Plasmidtyp. Ihre vorteilhaften Eigenschaften kommen vor allem in Stämmen zur Geltung, die wie E.coli SB3 (siehe weiter unten) aufgrund ihrer besonderen Eigenschaften eine hohe Produktion von L-Prolin ermöglichen. So unterscheiden sich die maximalen Prolinkonzentrationen und die Produktivitäten bei dem Stamm E.coli SB2, der nur eine mittlere Überproduktion ermöglicht, für die Plasmide pSB122, pSB124 und pSB126 nur geringfügig voneinander (Tabelle 1). In E.coli SB3 dagegen ergeben pSB 122 und pSB126 erheblich bessere Werte als pSB124 (vgl. z.B. Tabelle 2 in Beispiel 4; für pSB123 wurden die gleichen Werte wie für pSB122 gefunden). Offenbar produziert das konstitutive chromosomale Gen proC in E.coli SB3 nicht mehr genügend 1-Pyrrolin-5-carboxylat-Reductase, um die von den im Überschuß vorhandenen ersten beiden Enzymen dieses Stoffwechselweges gebildete Zwischenstufe (1-Pyrrolin-5-carboxylat) rasch genug in L-Prolin umzuwandeln.

Die Erfindung umfaßt somit auch Plasmide, die neben den Genen proA und proB (mutiert) auch noch das Gen proC auf ihrer DNS-Sequenz enthalten.

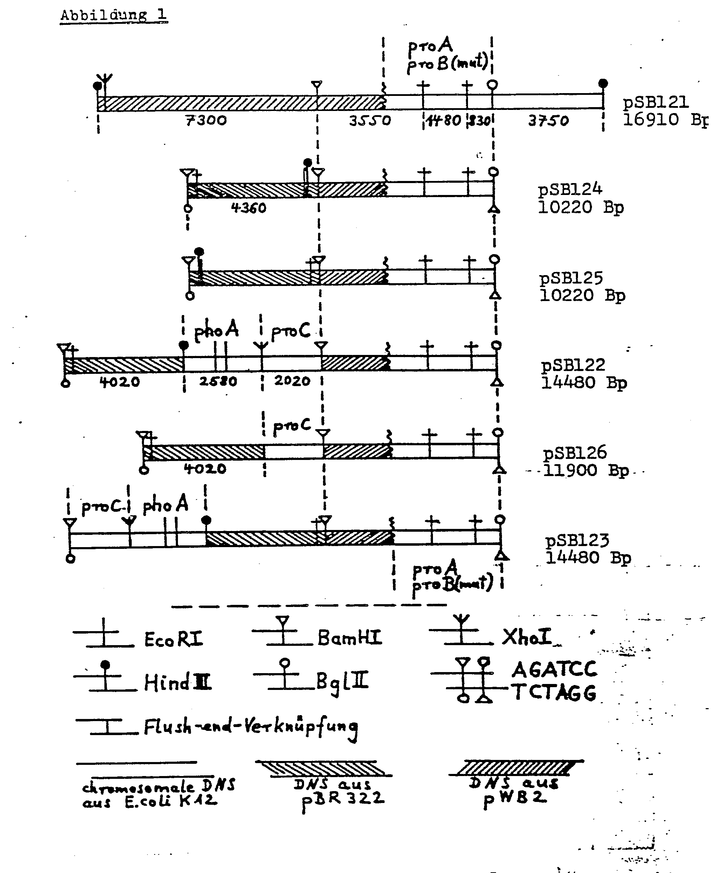

Die Methoden zur in vitro Neukombination und zur Klonierung von DNS sind in verschiedenen Publikationen zusammenfassend beschrieben worden, z.B. in Methods in Enzymol., Vol. 68, Ray Wu (Ed.), Academic Press, New York 1979. Eine Zusammenstellung der im Rahmen dieser Erfindung in den Beispielen 1 und 2 hergestellten neukombinierten Plasmide enthält die Abb. 1, in der die ringförmigen Strukturen in einer linearisierten Form dargestellt sind. Die Wellenlinie kennzeichnet den Übergang von chromosomaler DNS zu Vektor-DNS aus pWB2. Dieser ist weniger als 1400 Bp von der rechts daneben gelegenen SalI-Sequenz entfernt, aber nicht genau definiert.

4

Die Sequenz $\frac{AGATCC}{TCTAGG}$ ist durch kovalente Verknüpfung eines BamHI-Einzelstrangendes mit einem BglII-Einzelstrangende entstanden und ist durch keine der beiden genannten Restriktionsendonucleasen spaltbar.

Die Erfindung beinhaltet auch die Verfahren zur Herstellung der genannten Plasmide. Diese Verfahren bestehen darin, daß man chromosomale DNS-Fragmente mit den Genen proA und proB aus einer antimetabolitresistenten E.coli-Mutante cloniert, oder daß man durch in-vitro Neukombination ein Fragment mit den Genen proA und proB mit einem Fragment mit dem Gen proC auf einem Plasmid vereinigt.

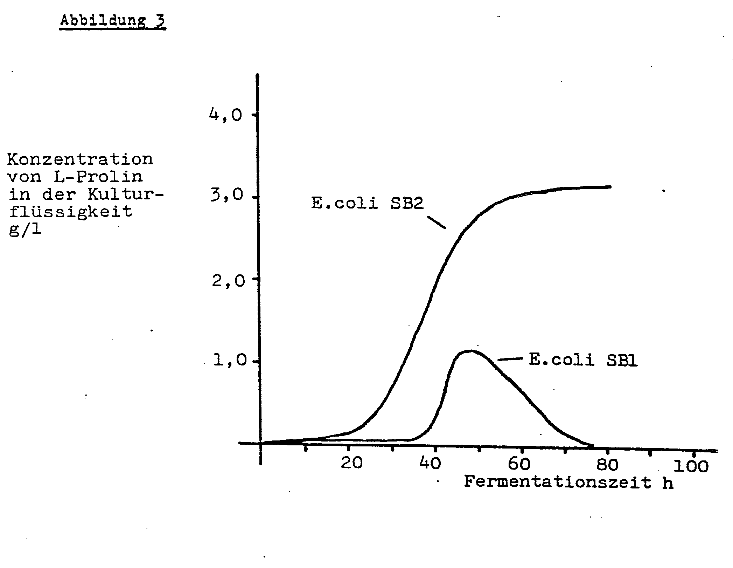

Ein weiterer Gegenstand der vorliegenden Erfindung ist die Verbesserung von antimetabolitresistenten E.coli-Mutanten durch Einführung weiterer chromosomaler Mutationen, die unabhängig vom Vorhandensein neukombinierter Plasmide der oben erwähnten Art eine Steigerung der L-Prolin-Ausscheidung bewirken. So wurde in den Stamm E.coli SB1 (Herstellung im Beispiel 1a beschrieben), aus dem auch die chromosomale DNS für die Herstellung des neukombinierten Plasmids pSB121 isoliert wurde, eine Mutation eingeführt, die das Wachstum mit L-Prolin als einziger Kohlenstoff- oder Stickstoffquelle verhindert. Wie aus Abb. 3 zu ersehen ist, liefert die so erhaltene Mutante E.coli SB2 ca. dreimal soviel L-Prolin wie E.coli SB1.

Die Ausbeute an L-Prolin ließ sich durch Einführung eines Stoffwechseldefektes, der das Zellwachstum begrenzt, noch weiter steigern. So ergaben einige Mutanten von E.coli SB2 mit Bedürftigkeiten für andere Aminosäuren und für Vorstufen der Nuleinsäuresynthese höhere Konzentrationen an L-Prolin. Als besonders geeignet erwies sich der Stamm E.coli SB3, der aus E.coli SB2 durch Einführung einer Bedürftigkeit für L-Leucin erhalten wurde, unter den in Beispiel 4 beschriebenen Bedingungen mit 60-100, insbesondere mit 75-85, vorzugsweise mit 80mg/l L-Leucin. Die Konzentration an L-Prolin nahm gegenüber E.coli SB2 um den Faktor 1,5-2 zu.

Die für das Wachstum einer Mangelmutante wie E.coli SB3 benötigte Aminosäure kann auch vorteilhaft in Form einer Aminosäuremischung zugesetzt werden. So ließ sich die maximale Prolinkonzentration und die Produktivität von E.coli SB3 mit pSB122 dadurch auf 13,8 g/l bzw. auf 0,21 g/l/h steigern, daß L-Leucin durch eine äquivalente Menge z.B. Casamino Acids ersetzt wurde.

Die Erfindung besteht damit auch in der Verwendung von E.coli-Mutanten, insbesondere den Mutanten E.coli SB2 und SB3 zur fermentativen Herstellung von L-Prolin. Damit sind gleichzeitig Verfahren zur Herstellung neuer Bakterienstämme und Verfahren zur Herstellung von L-Prolin mit Hilfe dieser Stämme gemeint (Ansprüche 17-22).

Es ist bekannt, daß Bakterien solche Plasmide besonders leicht verlieren, die in hohem Maße eine unnatürliche Stoffwechselleistung induzieren, z.B. die Überproduktion eines Enzyms oder einer Aminosäure /vgl. z.B. T. Imanaka et al., J. Gen Microbiol. 118, 253-261 (1980)/. Dies trifft insbesondere auch für die Plasmide pSB122 und pSB123 zu. So sind am Ende der Fermentationen von E.coli SB2 (pSB122) und E.coli SB3 (pSB122) unter den in Beispiel 3 und 4 beschriebenen Bedingungen 20-25 % bzw. mehr als 90 % der Zellen plasmidfrei (vgl. in den Tabellen 1 und 2 die Werte für den Anteil an ampicillinresistenten, d.h. plasmidhaltigen Zellen an der Gesamtpopulation).

Eine wesentliche Verbesserung der Stabilität von pSB122 ließ sich durch Verkleinerung des Moleküls um ca. 2580 BP erreichen, wobei das Plasmid pSB126 resultierte (Beispiel 2).
pSB126 ist auch in E.coli SB3 unter den in Beispiel 4 beschriebenen Bedingungen stabil und ergibt entsprechend eine höhere Prolinkonzentration sowie eine bessere Produktivität als pSB122 in dem gleichen Stamm (Tabelle 2), während umgekehrt in

6

E.coli SB2 pSB122 die besseren Resultate lieferte (Tabelle 1).

Eine Plasmidstabilisierung wird auch erreicht, wenn man Bedingungen schafft, unter denen nur die plasmidhaltigen Zellen wachsen können. Dies war bei den hier beschriebenen Plasmiden durch Zusatz des Antibiotikums Ampicillin zum Medium möglich, da sie in ihren Wirtszellen Resistenz gegen Ampicillin bewirkten. So ließen sich die maximalen Prolinkonzentrationen der Stämme E.coli SB2 (pSB122) und E.coli SB3 (pSB122) von 6,0 g/l auf 6,8 g/l bzw. von 12,5 g/l auf 16,1 g/l durch Zusatz von 100 mg/l Ampicillin zum Fermentationsmedium steigern (Vergleich der Ergebnisse in Beispiel 5 mit den entsprechenden Werten in den Tabellen 1 und 2).

Alle bekannten Verfahrensschritte wurden nicht im einzelnen ausgeführt, da sie bereits ausführlich in der Europäischen Patentanmeldung 0023882 genannt worden sind.

Am 8. Juli 1981 wurden bei der "Deutschen Sammlung von Mikroorganismen (DSM)" in Göttingen, Bundesrepublik Deutschland, folgende Mikroorganismen hinterlegt (Hinterlegungsnummer):

| | |
|---|---|
| Escherichia coli SB1 | (DSM 2120) |
| Escherichia coli SB2 | (DSM 2121) |
| Escherichia coli SB3 | (DSM 2122) |
| E.coli SB2 mit pSB121 | (DSM 2123) |
| E.coli SB3 mit pSB122 | (DSM 2124) |

Für folgende in dieser Anmeldung genannten Mikroorganismen und Plasmide liegen bereits Hinterlegungen vor:

| | |
|---|---|
| E.coli SB44 | (DSM 1606, 16.7.79) |
| E.coli HB 101 | (DSM 1607, 16.7.79) |
| Plasmid pWB2 | (ATCC 40013, 16.7.79) |
| Plasmid pBR322 | (ATCC 40015, 16.7.79) |
| Plasmid pSB53 | (ATCC 40020, 16.7.79) |

BAD ORIGINAL

7

Der Stamm E.coli X474 wurde von P.Broda (England) bezogen, der Stamm E.coli LEP1 von E. Yagil (Tel Aviv) und von CGSC (Yale Univ., New Haven, USA).

8

**Beispiel 1:**

**Herstellung von Plasmiden mit den Genen pro A und pro B (mut)**

a)  Isolierung der antimetabolitresistenten Mutante
    E.coli SB1

Je 0,1 ml einer Übernachtkultur von E.coli K12 Wildtyp
in E-Medium /H.J. Vogel und D.M. Bonner, J.Biol. Chem.
**218**, 97-106 (1956)/ wurden auf Agarplatten (1,5% Agarose
in E-Medium) ausplattiert, welche 15µg/ml D/L-Dehydroprolin (bezogen von der Fa. Calbiochem GmbH, 6300 Giessen,
FRG) enthielten.  Nach 24stündiger Inkubation waren durch
Spontanmutation entstandene, gegen Dehydroprolin resistente Einzelkolonien in einer Häufigkeit von ca. 1 Kolonie
pro $3 \times 10^6$ Wildtypzellen gewachsen.  Diese wurden mit
Hilfe von sterilen Zahnstochern auf Agarplatten mit Minimalmedium (M9-Medium mit 2 g/l Glucose, 20 mg/l L-Tryptophan und 40 mg/l Adenin;vgl. R.W. Davis et al., Advanced
Bacterial Genetics, Cold Spring Harbor Laboratory, New
York 1980, S. 203) überführt, auf welche als Indikatorstamm zuvor $1 \times 10^6$ Zellen pro Platte der prolinbedürftigen Mutante E.coli LEP1 /proC, purE, trpE; vgl. M. Bracha
und E. Yagil, Molec. gen. Genet. **122**, 53-60 (1973)/ ausplattiert worden waren.  Nach 24stündiger Inkubation bei
$37^o$C liessen sich L-Prolin ausscheidende Kolonien durch
einen Hof von Mikrokolonien des Indikatorstammes identifizieren.  Eine dieser L-Prolin produzierenden Kolonien
wurde für die weiteren Versuche abgeimpft und erhielt die
Bezeichnung E.coli SB1.  Der Stamm erwies sich auch als
resistent gegen den Antimetaboliten Acetidin-2-carbonsäure.

b)  Herstellung des Plasmids pSB121

Als Vektor wurde das Plasmid pWB2 verwendet, welches eine Schnittstelle für die Restriktionsendonuklease Hind
III besitzt und Resistenz gegen Ampicillin und Colicin El vermittelt /W.Boidol et al., Molec. gen. Genet. 152, 231-237 (1977)_7. Alle Plasmid-DNS wurde nach einem Verfahren von G.O. Humphreys et al., Biochem. Biophys. Acta 383, 457-463 (1975) isoliert und gegen DNS-Puffer (45 mM TrisHCl, 0,1 mM EDTA, pH 7,9) dialysiert. Hochmolekulare chromosomale DNS aus E.coli SB1 wurde nach dem Phenolextraktionsverfahren von H.Saito und K. Miura, Biochem. Biophys. Acta 72, 619-629 (1963), jedoch unter Auslassung der RNAse-Verdauung isoliert und ebenfalls gegen DNS-Puffer dialysiert.

pWB2 und chromosomale DNS aus E.coli SB1 wurden mit der Restriktionsendonuklease HindIII umgesetzt. Die Reaktionsmischung enthielt 80µl chromosomale DNS (140 µg/ml), 20 µl pWB2 (300 µg/ml), 75 µl Reaktionspuffer (20 mM TrisHCl, 20 mM Mercaptoäthanol, 20 mM $MgCl_2$, 180 mM NaCl, pH 7,4) und 50 µl einer verdünnten HindIII-Lösung mit einer zur quantitativen Umsetzung ausreichenden Enzymkonzentration, welche in Vorversuchen ermittelt worden war. Die Mischung wurde 75 Min. bei $37^o$C und anschliessend zur Inaktivierung des Enzyms 10 Min. bei $65^o$C gehalten. Zu 180 µl der HindIII-Umsetzung wurden 25 µl Ligase-Puffer (660 mM TrisHCl, 66mM $MgCl_2$, 100 mM Dithiothreitol, pH 7,6), 25 µl ATP (4 mM), 15 µl $H_2O$ und 5 µl T4-Ligase (100 Einheiten/ml; Biolabs, Beverly, Md. USA) gegeben. Die Mischung wurde 17 Std. bei $13^o$C inkubiert, zweimal mit je 250 µl Phenol, welches zuvor mit TES-Puffer (50 mM TrisHCl, 50 mM NaCl, 5 mM EDTA, pH 8,0) gesättigt worden war und dreimal mit je 250 µl Äther extrahiert. Die DNS wurde anschliessend in der üblichen Weise mit Äthanol gefällt und in DNS-Puffer gelöst.

Als Empfängerstamm für die Klonierung diente der gut transformierbare, prolinbedürftige Stamm E.coli HB101 (proA, leuB, thi, hsdR, hsdM, recA; vgl. R.W. Davis et al., Advanced Bacterial Genetics, Cold Spring Harbor Laboratory, New York 1980, S.7). Die Zellen wurden nach einem Verfahren von M. Dagert und S.D. Ehrlich, Gene 6, 23-28 (1979) mit der DNS aus der Ligase-Umsetzung transformiert. Nach der Transformation wurde die Zellsuspension (0,6 ml) mit 8 ml L-Medium /E.S.Lennox, Virology 1, 190 - 206 (1955)7 verdünnt und 1 Std. bei 37°C inkubiert. Nach Zugabe weiterer 100 ml L-Medium und 50 µg/ml Ampicillin wurde über Nacht bei 37°C inkubiert. Die Zellsuspension wurde auf M9-Agar mit 2 g/l Glucose, 20 mg/l L-Leucin und 1 mg/l Vitamin B1 ausplattiert. Nach 48 Std. bei 37°C waren einzelne, prolinprototrophe Kolonien gewachsen.

Aus einigen dieser Kolonien, für welche sich in einem Fütterungsversuch mit dem prolinbedürftigen Indikatorstamm E.coli LEP1 wie in Beispiel 1a eine Ausscheidung von L-Prolin nachweisen liess, wurde Plasmid-DNS isoliert. Bei der Analyse durch Agarose-Gelelektrophorese erwiesen sich diese Isolate als uneinheitlich, was auf eine Instabilität des klonierten DNS-Fragments hinwies. Durch erneute Transformation und Selektion von ampicillinresistenten und prolinprototrophen Zellen wurde schliesslich ein Klon erhalten, aus dem sich ein einheitliches, stabiles Plasmid isolieren liess, welches als pSB121 bezeichnet wurde.

Eine Restriktionskarte von pSB121 ist in Abb. 1 dargestellt. Sie weist nur eine HindIII-Sequenz auf und enthält nicht mehr den gesamten Vektor. Dies weist darauf hin, dass aus der primär gebildeten neukombinierten DNS ein Teilbereich abgespalten wurde, der sowohl Sequenzen des Vektors als auch des klonierten chromosomalen Fragments und die zwischen beiden liegende HindIII-Sequenz umfasst.

Durch Transformation mit pSB121 wurde die proB-Mutante E.coli X474 /P. Broda, J. Bacteriol. 117, 741 - 746 (1974)7 prolinprototroph, woraus hervorgeht, dass pSB121 neben proA auch das dicht benachbarte Gen proB
trägt. Ein Fütterungsversuch wie oben beschrieben·
zeigt, dass der Stamm E.coli HB101 (pSB121) L-Prolin
produziert, währen E.coli HB101 ohne Plasmid kein L-Prolin ausscheidet.

c) Herstellung der Plasmide pSB124 und pSB125

pSB121 wurde durch Umsetzung mit den Restriktionsendonukleasen BamHI und BglII in zwei Fragmente mit den Molekulargewichten 5860 Bp und 11050 Bp zerlegt, das kleinere Fragment wurde durch präparative Gelelektrophorese
in 0,7 % Agarose und Elektroelution (H.O. Smith, in
Methods in Enzymology, Vol. 65, Academic Press, New York,
1980, S. 371) aus der Mischung abgetrennt. Das Vektorplasmid pBR322 /F. Bolivar et al., Gene 2, 95 - 113
(1977)7 wurde durch Umsetzung mit BamHI linearisiert.

2,5 µg lineares pBR322 und 6 µg des BamHI/BglII-Fragments.
von 5860 Bp aus pSB121 wurden wie in Beispiel 1b durch
Umsetzung mit T4-Ligase kovalent verknüpft. Dies ist
möglich, da durch BamHI und BglII erzeugte Einzelstrangenden die gleiche Sequenz haben. Durch Transformation
von E.coli HB101 mit der DNS aus der Ligase-Umsetzung und
Selektion von ampicillinresistenten und prolinprototrophen Zellen wie in Beispiel 1b wurden mehrere Zellklone
erhalten, aus denen sich zwei als pSB124 und pSB125 bezeichnete Plasmide isolieren liessen. Beide enthalten
je ein Molekül des Fragments aus pSB121 und des mit
BamHI linearisierten pBR322 und unterscheiden sich nur
durch die Orientierung der beiden Fragmente zueinander.
Restriktionskarten von pSB124 und pSB125 sind in ABB. 1
dargestellt. Sie weisen nur eine BamHI- und keine BglII-
Sequenz auf, da sich die durch Verknüpfung eines BamHImit einem BglII-Einzelstrangende entstandene Sequenz
durch keines der beiden Enzyme wieder spalten lässt.

12

Die Stämme E.coli HB101 (pSB124) und E.coli HB101 (pSB125) sind beide in der Lage, L-Prolin auszuscheiden, wie sich durch einen Fütterungsversuch in Beispiel 1a zeigen liess. Dies beweist, dass die Gene proA und proB(mut) auf dem BamHI/BglII-Fragment von 5860 Bp in pSB121 lokalisiert sind.

Beispiel 2:

Herstellung von Plasmiden mit den Genen proA, proB(mut) und proC

a) Herstellung der Plasmide pSB122 und pSB123

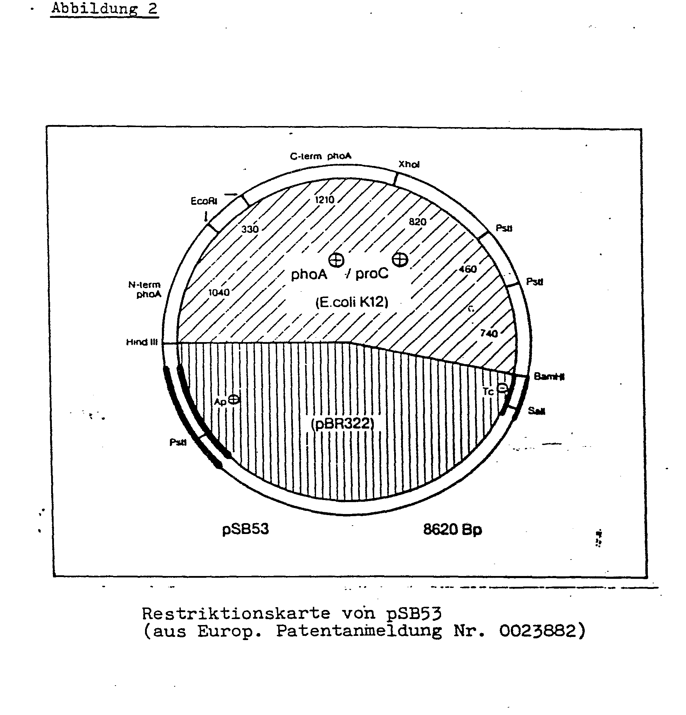

pSB53, welches die Gene phoA und proC auf einem chromosomalen HindIII/BamHI-Fragment aus E.coli K12 von 4600 Bp trägt, ist in der Europäischen Patentanmeldung Nr. 0023882 beschrieben worden. Eine Restriktionskarte des Plasmids ist in Abb. 2 dargestellt. Das Gen proC befindet sich zwischen der XhoII- und der BamHI-Sequenz auf einem Fragment von 2020 Bp.

pSB122 und pSB123 wurden durch Insertion des gleichen BamHI/BglII-Fragments von 5860 Bp, welches in Beispiel 1c zur Herstellung von pSB124 und pSB125 verwendet wurde und welches die Gene proA und proB(mut) trägt, in die BamHI-Sequenz von pSB53 in den beiden möglichen Orientierungen erhalten.

Zu einer Lösung von 12 µg pSB53, welches mit BamHI linearisiert worden war, und 9 µg pSB121, welches mit BamHI und BglII gespalten worden war, in 50 µl DNS-Puffer wurden 10 µl Ligase-Puffer (vgl. Beispiel 1b), 10 µl ATP (4 mM), 25 µl Wasser und 5 µl T4-Ligase gegeben. Die Mischung wurde wie in Beispiel 1b inkubiert und aufgearbeitet. Als Wirt für die Transformation diente der Stamm E.coli SB44, eine phosphatasennegative (phoA)-Mutante von E.coli HB101. Dieser Stamm sowie ein Verfahren zur Selektion von phosphatasepositiven Zellen sind ebenfalls in

13

der Europäischen Patentanmeldung Nr. 0023882 beschrieben.

Nach der Transformation von E.coli SB 44 wurden ampicillinresistente, prolinprototrophe und phosphatasepositive Zellen selektioniert. Aus diesen liessen sich die Plasmide pSB122 und pSB123 isolieren, deren Restriktionskarte in Abb. 1 dargestellt ist.

Durch Transformation der proB und proC-Mutanten E.coli X474 und E.coli LEP1 liess sich zeigen, dass pSB122 und pSB123 neben proA auch die Gene proB und proC tragen. Alle Stämme mit pSB122 oder pSB123 ergaben ein positives Ergebnis in dem in Beispiel 1a beschriebenen Fütterungs-test und sind daher in der Lage, L-Prolin auszuscheiden.

b) Herstellung von pSB126

pSB126 ist aus pSB122 durch Eliminierung des HindIII/ XhoI-Fragments von 2580 Bp, welches das Gen phoA trägt, entstanden. pSB122 wurde durch Umsetzung mit HindIII und XhoI in zwei Fragmente von 2580 und 11900 Bp zerlegt, das grössere Fragment wurde durch präparative Gelelektrophorese und Elektroelution wie in Beispiel 1c abge-trennt und isoliert.

Die Einzelstrangenden dieses Fragments wurden durch Umsetzung mit T4-Polymerase (11000 Einheiten/ml P.L. Biochemicals Inc., Milwaukee, Wis., USA) zu Doppelstrangenden aufgefüllt. Die Reaktionsmischung enthielt 50 µl einer Lösung von 8 µg des XhoI/HindIII-Fragments von 11900 Bp, 10 µl Ligase-Puffer (vgl. Beispiel 1b), je 5 µl von 1 mM Lösungen der 4 Desoxynukleosid-triphosphate dATP, dGTP, dTTP und dCTP, 10 µl Wasser und 10 µl einer Verdünnung von T4-Polymerase mit 2,2 Einheiten. Die Mischung von insgesamt 100 µl wurde 30 Min. bei 37$^o$C und zur Inaktivierung der Polymerase 10 Min. bei 65$^o$C gehal-ten. Danach wurden 5 µl Ligase-Puffer, 15 µl ATP (4mM), 10 µl Wasser und 20 µl T4-Ligase zugefügt. Die Mischung

14

wurde wie in Beispiel 1b inkubiert und aufgearbeitet. Mit der DNS wurde E.coli SB44 transformiert, aus dem Transformationsansatz wurden ampicillinresistente, prolinprototrophe, phosphatasenegative Zellen selektioniert. Aus diesen liess sich das Plasmid pSB126 isolieren, dessen Restriktionskarte in Abb. 1 dargestellt ist.

In der gleichen Weise wie in Beispiel 2a beschrieben, wurde für pSB126 bewiesen, dass es ausser proA auch proB und proC besitzt und eine Überproduktion von L-Prolin bewirkt.

Beispiel 3:

Isolierung der Mutante E.coli SB2 und ihre Verwendung zur Produktion von L-Prolin in Verbindung mit Plasmiden aus Beispiel 1 und 2

a) Isolierung von E.coli SB2

E.coli SB2 wurde aus der antimetabolitresistenten Mutante E.coli SB1 (Beispiel 1) durch Mutagenese mit 1-Nitroso-3-nitro-1-methyl-guanidin (NNMG) und Selektion von Zellen, die L-Prolin weder als Kohlenstoff- noch als Stickstoffquelle verwerten können, erhalten. Dazu wurde in bekannter Weise /E.A. Adelberg et al., Biochem. Biophys.Res. Comm. 18, 788 - 795 (1965)/ eine Suspension von $10^9$ Zellen/ml E.coli SB1 in einer Lösung von 9g/l NaCl durch 10minütige Einwirkung von 10 µg/ml NNMG mutagenisiert. Danach wurde überschüssiges Mutagen durch Waschen mit steriler NaCl-Lösung entfernt, die Zellen wurden in einer geeigneten Verdünnung auf M9-Agar mit 2 g/l Glucose als C-Quelle ausplattiert und bei 37°C bis zur Bildung von Einzelkolonien inkubiert. Mit Hilfe der Stempeltechnik wurden die Kolonien sowohl auf M9-Agar mit 2,5 g/l L-Prolin anstelle von Glucose (L-Prolin als einzige C-Quelle) als auch auf M9-Agar mit 2 g/l Glucose und 2,5 g/l L-Prolin anstelle von $NH_4Cl$ (L-Prolin als einzige N-Quelle) überführt. Es liessen sich mehrere Kolonien identifizieren, die auf beiden prolinhaltigen Medien nicht wuchsen. Eine von diesen wurde unter der Bezeichnung E.coli SB2 für die weiteren Versuche eingesetzt.

b) Fermentative Herstellung von L-Prolin

Durch Transformation von E.coli SB2 mit den Plasmiden pSB121, pSB122, pSB124 und pSB126 und Selektion von ampicillinresistenten Kolonien wurden die entsprechenden plasmidhaltigen E.coli-Stämme erhalten.

Ausgehend von einer frischen Einzelkolonie eines solchen Stammes auf L-Agar mit 100 mg/l Ampicillin werden 20 ml L-Medium, welchem 100 mg/l Ampicillin zugefügt sind, beimpft. Die Kultur (1. Vorzucht) wird über Nacht bei 37°C geschüttelt und dann zur Beimpfung von 500 ml M9-Medium mit 5 g/l Glucose, 1 g/l Casamino Acids (Difco) und 100 mg/l Ampicillin eingesetzt, welches 6 - 7 Std. bei 37°C geschüttelt wird (2.Vorzucht).

In einem 20 l-Glasfermenter, welcher eine sterile Lösung von 70 g $Na_2HPO_4.2H_2O$, 30 g $KH_2PO_4$, 10 g $NH_4Cl$ und 5 g NaCl in 8 l Wasser enthält, werden 1 Liter einer sterilen Lösung von 500 g/l Glucose sowie 1 Liter einer sterilen Lösung, welche 200 g/l Mononatrium-L-glutamat, 2,5 g/l $MgSO_4 \cdot 7H_2O$ und 10 mg/l Vitamin B1 enthält, gegeben. Der Fermenter wird mit der 2. Vorzucht beimpft und bei 37°C, 0,7 atü Druck und einer Belüftung von 10 l/min. mit 220 u/min. bis zum Erreichen der maximalen Prolinkonzentration (100 - 200 Std.) gerührt. Der pH-Wert wird durch laufende Zugabe einer 12%igen $NH_3$-Lösung mit Hilfe einer automatischen pH-Steuerung auf 6,5 gehalten.

In regelmässigen Abständen von 3 bis 4 Stunden werden Proben entnommen, in denen der Gehalt an L-Prolin nach einer Methode von F. P.Chinard, J. Biol. Chem.199, 91 (1952) bestimmt wird, welche auf der Reaktion von L-Prolin

16

mit Ninhydrin in essigsaurer Lösung beruht (vgl. R.H. Abeles, in Methods in Enzymol. Vol. XVII B, Academic Press, New York 1971). Zur Bestätigung wird in einem kleinen Teil der Proben der Prolingehalt durch einen quantitativen mikrobiologischen Test mit Hilfe der proC-Mutante E.coli LEP1 gemessen. In einigen Proben aus der Schlussphase der Fermentation wird ausserdem das Verhältnis von ampicillinresistenten Zellen zur Gesamtkeimzahl als Mass für den Plasmidverlust ermittelt. Die plasmidfreien Stämme E.coli SB2 werden in der gleichen Weise fermentiert mit dem einzigen Unterschied, dass die Vorzuchtmedien ampicillinfrei sind.

Die Ergebnisse dieser Fermentationen sind in Abb. 3 (plasmidfreie Stämme) und in Tabelle 1 zusammengefasst.

TABELLE 1

Ergebnisse der fermentativen Herstellung von L-Prolin mit E.coli SB2 und plasmidhaltigen Abkömmlingen dieses Stammes in M9-Medium mit 50 g/l Glucose und 20 g/l Mononatrium-L-glutamat, pH 6,5

| Stamm (Plasmid) | Maximale Konzentration von L-Prolin $c_{max}$(g/l) | Fermentationszeit $t_{90}$(h) * | Produktivität $c_{90}/t_{90}$ (g/l/h) * | ampicillin-resistente Zellen bei Fermentationsende |
|---|---|---|---|---|
| SB2 | 3,2 | 51 | 0,056 | – |
| SB2 (pSB121) | 4,1 | 46 | 0,080 | >90 % |
| SB2 (pSB122) | 6,0 | 49 | 0,110 | 75 – 80 % |
| SB2 (pSB124) | 5,6 | 56 | 0,090 | 100 % |
| SB2 (pSB126) | 5,3 | 51 | 0,094 | 100 % |

\* $c_{90}$ und $t_{90}$: Prolinkonzentration und Fermentationszeit bei Erreichen von 90 % der maximalen Prolinkonzentration

Beispiel 4:

**Isolierung der Mutante E.coli SB3 und ihre Verwendung
zur Herstellung von L-Prolin nach Transformation mit
den Plasmiden pSB122, pSB124 und pSB126**

a)  **Isolierung von E.coli SB3**

Als Ausgangsstamm diente der Stamm E.coli SB2, welcher in
der in Beispiel 3a beschriebenen Weise mit NNMG mutagenisiert und gewaschen wurde.  Danach wurden die Zellen in
einer geeigneten Verdünnung auf M9-Agar mit 2,0 g/1 Glucose und 20 mg/1 L-Leucin ausplattiert und bei 37°C bis
zur Bildung von Einzelkolonien inkubiert.  Durch Stempeln
auf M9-Agar mit 2,0 g/1 Glucose ohne Aminosäurezusatz
konnten einige Kolonien mit einer Bedürftigkeit für L-
Leucin identifiziert werden.  Eine von diesen wurde unter
der Bezeichnung E.coli SB3 für die folgenden Versuche eingesetzt.  Durch Überführung auf Agarmedien mit L-Prolin
als einziger C- oder N-Quelle wie in Beispiel 3a liess
sich zeigen, dass E.coli SB3 wie E.coli SB2 L-Prolin nicht
als Kohlenstoff- oder Stickstoffquelle verwerten kann.

b)  Fermentative Herstellung von L-Prolin

Durch Transformation mit den Plasmiden pSB122, pSB124
und pSB126, wie in Beispiel 3b, wurden die entsprechenden,
von E.coli SB3 abgeleiteten plasmidhaltigen Stämme hergestellt.  Die Fermentationen wurden unter den gleichen
Bedingungen ausgeführt wie in Beispiel 3b mit dem einzigen Unterschied, dass den Medien für die Hauptfermentation 80 mg/1 L-Leucin zugesetzt wurde.  Die Vorzuchten
von plasmidfreiem E.coli SB3 erfolgten wiederum in Medien
ohne Ampicillin.  Die Ergebnisse dieser Versuche sind in
Tabelle 2 zusammengefasst.

TABELLE 2

Ergebnisse der Fermentation von E.coli SB3 und plasmidhaltigen Abkömmlingen dieses Stammes in M9-Medium mit
50 g/l Glucose, 20 g/l Mononatrium-L-glutamat und
80 mg/l L-Leucin, pH 6,5

| Stamm (Plasmid) | Maximale Konzentration von L-Prolin $c_{max}$(g/l) | Fermentations-zeit $t_{90}$(h) | Produktivität $c_{90}/t_{90}$ (g/l/h) | ampicillin-resistente Zellen bei Fermenta-tionsende |
|---|---|---|---|---|
| SB3 | 5,5 | 77 | 0,064 | - |
| SB3 (pSB122) | 12,5 | 74 | 0,152 | <10 % |
| SB3 (pSB124) | 12,1 | 67 | 0,163 | 75 % |
| SB3 (pSB126) | 14,0 | 66 | 0,191 | >80 % |

Beispiel 5:

**Fermentative Herstellung von L-Prolin unter Zusatz von Ampicillin**

Die Stämme E.coli SB2 (pSB122) und E.coli SB3 (pSB122)
wurden wie in Beispiel 3 b) bzw. 4 b) fermentiert mit
dem einzigen Unterschied, dass auch dem Medium der Hauptfermentation 100 mg/l Ampicillin zugesetzt wurde.

Es ergaben sich die folgenden Werte für die maximale Konzentration an L-Prolin ($c_{max}$), die Fermentationszeit bis
zum Erreichen von 90% der maximalen Konzentration ($t_{90}$)
und für die aus den beiden Werten berechnete Produktivität
($c_{90}/t_{90}$):

| | $c_{max}$(g/l) | $t_{90}$(h) | $c_{90}/t_{90}$ (g/l/h) |
|---|---|---|---|
| E.coli SB2 (pSB122) | 6,8 | 51 | 0,120 |
| E.coli SB3 (pSB122 | 16,1 | 93 | 0.156 |

Beispiel 6:

<u>Fermentation von E.coli SB3 (pSB122) in Medium mit</u>
<u>Casamino Acids anstelle von L-Leucin</u>

Der Stamm wurde wie in Beispiel 4 b) fermentiert mit dem einzigen Unterschied, dass das Medium der Hauptfermentation 3,5 g/l Casamino Acids anstelle von 80 mg/l L-Leucin enthielt.

Die folgenden Werte für die maximale Prolinkonzentration $c_{max}$, die Fermentationszeit $t_{90}$ und die Produktivität $c_{90}/t_{90}$ wurden ermittelt:

$$c_{max}: \quad 13,8 \text{ g/l}$$
$$t_{90}: \quad 59 \text{ h}$$
$$c_{90}/t_{90}: \quad 0,211 \text{ g/l/h}$$

1

Patentansprüche

1) Plasmide, die eine DNS-Sequenz mit den Genen proA und proB enthalten, wobei proB in einer mutierten Form vorliegt, die eine antimetabolitresistente γ-Glutamyl-Kinase codiert.

2) Plasmid pSB 121
3) Plasmid pSB 124
4) Plasmid pSB 125

5) Verfahren zur Herstellung von Plasmiden entsprechend Anspruch 1, dadurch gekennzeichnet, daß man chromosomale DNS-Fragmente mit den Genen proA und proB für die Biosynthese von L-Prolin aus einer antimetabolitresistenten E.coli-Mutante cloniert.

6) Plasmide, die eine DNS-Sequenz mit den Genen proA, proB (mutiert) und proC enthalten, mit proB in einer mutierten Form entsprechend Anspruch 1.

7) Plasmid pSB 122
8) Plasmid pSB 123
9) Plasmid pSB 126

10) Verfahren zur Herstellung von Plasmiden entsprechend Anspruch 6, dadurch gekennzeichnet, daß man durch in-vitro-Neukombination ein Fragment mit den Genen proA und proB entsprechend Anspruch 1 mit einem Fragment mit dem Gen proC auf einem Plasmid vereinigt.

11) Mutante E.coli SB 1

12) Verwendung der Mutante E.coli SB 1 zur Herstellung des Plasmids pSB 121

13) Mutante E.coli SB 2

BAD ORIGINAL

2

14) Verwendung der Mutante E.coli SB 2, die Plasmide nach Anspruch 1-4 oder 6-9 enthält, zur fermentativen Herstellung von L-Prolin.

15) Mutante E.coli SB 3

16) Verwendung der Mutante E.coli SB 3, die Plasmide nach Anspruch 1-4 oder 6-9 enthält, zur fermentativen Herstellung von L-Prolin.

17) Verfahren zur Herstellung neuer Bakterienstämme, die Plasmide mit den Genen proA und proB (mutiert) enthalten, dadurch gekennzeichnet, daß man kompetente Zellen des betreffenden Stammes mit der Plasmid-DNS unter Transformationsbedingungen inkubiert und aus der Zellpopulation Antibiotika-resistente Zellen isoliert.

18) Verfahren zur Herstellung neuer Bakterienstämme, die Plasmide mit den Genen proA; proB (mutiert) und proC enthalten, dadurch gekennzeichnet, daß man kompetente Zellen des betreffenden Stammes mit der Plasmid-DNS unter Transformationsbedingungen inkubiert und aus der Zellpopulation Antibiotika-resistente Zellen isoliert.

19) Verfahren zur Herstellung Plasmid-haltiger E.coli SB 2-Stämme nach Anspruch 17.

20) Verfahren zur Herstellung Plasmid-haltiger E.coli SB 3-Stämme nach Anspruch 18.

21) Verfahren zur Herstellung von L-Prolin mit Hilfe von E.coli-Mutanten wie E.coli SB2 enthaltend Plasmide wie pSB121, pSB122, pSB124 oder pSB126, dadurch gekennzeichnet, daß man die Stämme in einem Glutaminsäure-haltigen Mineralsalzmedium fermentiert und das gebildete L-Prolin nach bekannten Verfahren isoliert.

3

22) Verfahren zur Herstellung von L-Prolin mit Hilfe von E.coli-Mutanten wie E.coli SB3 enthaltend Plasmide wie pSB122, pSB124 oder pSB126, dadurch gekennzeichnet, daß man die Stämme in einem Glutaminsäure-haltigen Mineralsalzmedium unter Zusatz von L-Leucin fermentiert und das gebildete L-Prolin nach bekannten Verfahren isoliert.

Abbildung 1

Abbildung 2

Restriktionskarte von pSB53
(aus Europ. Patentanmeldung Nr. 0023882)

Abbildung 3

Konzentration
von L-Prolin
in der Kulturflüssigkeit
g/l

4,0

3,0

2,0

1,0

E.coli SB2

E.coli SB1

20    40    60    80    100

Fermentationszeit h